**Europäisches Patentamt**

(19) **European Patent Office**

**Office européen des brevets**

(11) Publication number: **0 115 777**

**B1**

(12) **EUROPEAN PATENT SPECIFICATION**

(45) Date of publication of patent specification: **05.04.89**

(51) Int. Cl.[4]: **C 12 Q 1/68**

(21) Application number: **84100148.0**

(22) Date of filing: **09.01.84**

(54) **Method for determination of base sequence of DNA or DNA fragment.**

(30) Priority: **08.01.83 JP 1325/83**
**08.01.83 JP 1335/83**

(43) Date of publication of application:
**15.08.84 Bulletin 84/33**

(45) Publication of the grant of the patent:
**05.04.89 Bulletin 89/14**

(84) Designated Contracting States:
**CH DE FR LI NL SE**

(56) References cited:
**US-A-4 239 968**
**US-A-4 395 486**

**NATURE, vol. 274, no. 5666, July 1979, pages
87-89, Macmillan Journals Ltd., Basingstoke,
GB; J. STANLEY et al.: "A different approach to
RNA sequencing"
Nucleic Acids, Res. (1977), pp. 2527-37
Methods in Enzymology (ed.L. Gressmann),
vol. 65, Academic Press, N.Y. (1980), pp.
544-45+560-61**

(73) Proprietor: **FUJI PHOTO FILM CO., LTD.
210 Nakanuma Minami Ashigara-shi
Kanagawa 250-01 (JP)**

(72) Inventor: **Shiraishi, Hisashi
c/o Fuji Photo Film Co., Ltd. No. 210, Nakanuma
Minami Ashigara-shi Kanagawa (JP)**

(74) Representative: **Patentanwälte Grünecker, Dr.
Kinkeldey, Dr. Stockmair, Dr. Schumann, Jakob,
Dr. Bezold, Meister, Hilgers, Dr. Meyer-Plath
Maximilianstrasse 58
D-8000 München 22 (DE)**

## Description

### Background of the invention
### Field of the invention

The present invention relates to a method for determination of base sequence of DNA or DNA fragment. More particularly, this invention relates to a method for sequencing DNA or DNA fragment utilizing the radiation image recording and reproducing method using a stimulable phosphor sheet.

### Description of prior arts

Autoradiography has been known as a method for obtaining locational information on radioactively labeled substances distributed in at least one dimensional direction to form a row on a support medium.

For instance, the autoradiography comprises steps of: labeling organism-originating biopolymers such as proteins or nucleic acids with a radioactive element; resolving the radioactively labeled biopolymers, derivatives thereof or cleavage products thereof (hereinafter referred to as radioactively labeled substances) on a gel support (support medium) through a resolving process such as electrophoresis to form a resolved pattern of the radioactively labeled substances (the resolved pattern is not visible); placing said support medium and a high-sensitivity type X-ray film together in layers for a certain period of time to expose the film and developing said film to give the autoradiograph of the resolved pattern as a visible image on the film; and obtaining the locational information on the radioactively labeled substances from said visible image. Further, the identification of the polymeric substances, determination of molecular weight of the polymeric substances and isolation of the polymeric substances are performed based on the obtained locational information.

The autoradiography has been effectively utilized for determining the base sequence of nucleic acids such as DNA (or DNA fragment, hereinafter "DNA" may be used to include both DNA and DNA fragment) or the like.

Maxam-Gilbert method and Sanger-Coulson method are known as methods for sequencing DNA utilizing the autoradiography. In these methods, the base sequence of DNA is determined by geniously utilizing the characteristic structure of DNA that DNA is in the form of a double helix structure, which consists of two chain molecules stabilized through hydrogen bonding between two bases on each chain molecule, and that the base, which comprises a part of constitutional unit of DNA, is limited to only four, which are adenine (A), guanine (G), cytosine (C), and thymine (T), and that the hydrogen bonding between each constitutional base unit comprises only two combinations, namely, G—C and A—T.

For instance, Maxam-Gipbert method is performed by the procedure described below.

A group containing a radioactive isotope of phosphorus (P) is attached to a chain molecule of DNA or a DNA fragment at one end to be sequenced to prepare a radioactively labeled DNA molecule, and then the radioactively labeled DNA molecule is specifically cleaved at the constitutional unit containing a certain base by a certain chemical reaction to obtain, for instance, the following four kinds of base specific cleavage products.

1) guanine specific cleavage products,
2) guanine specific cleavage products,
   +adenine specific cleavage products,
3) thymine specific cleavage products
   +cytosine specific cleavage products,
and
4) cytosine specific cleavage products.

Then, each of the cleavage products is resolved all together in parallel on the same gel support through gel electrophoresis to obtain a resolved pattern consisting of rows of the cleavage products respectively resolved in one dimensional directions (the pattern is not visible). The above-described autoradiography is then performed on the rows of the resolved cleavage products to obtain a visible image of the autoradiograph on a radiographic film.

In the case of employing the above-described set of cleavage products, the visualized autoradiograph shows the following four kinds of the resolved rows:

1) a resolved row indicating resolved positions of the guanine specific cleavage products,

2) a resolved row indicating resolved positions of both the guanine specific cleavage products and the adenine specific cleavage products,

3) a resolved row indicating resolved positions of both the thymine specific cleavage products and the cytosine specific cleavage products, and

4) a resolved row indicating resolved positions of the cytosine specific cleavage products.

In the sequencing method, for instance, the resolved row 1) and row 2) are compared therebetween to identify the resolved positions for both the guanine specific cleavage products and the adenine specific cleavage products, respectively. Further, comparison between the resolved row 3) and 4) is performed to identify the resolved positions for both the thymine specific cleavage products and the cytosine cleavage products, respectively.

Thus, the resolved positions are identified on all the base specific cleavage products, namely, the guanine (G) specific cleavage products, the adenine (A) specific cleavage products, the thymine (T) specific cleavage products and the cytosine (C) specific cleavage products, respectively. Since the migration distances of the cleavage products are in adversely proportional to the molecular weight thereof, all bases can be sequenced by identifying the positions of bases sequentially from the radioactively labeled end of the chain molecule.

Accordingly, the autoradiography for base sequencing of DNA using a radiographic film is advantageous in that the locational information on radioactively labeled substances can be observed visually.

However, the conventional autoradiography employing, for instance, the above-described set of the cleavage products, has a drawback. Specifically, achieving an image of the cleavage products by conventional autoradiography is rather time-consuming. Reduction of this time period can only be achieved by using highly labeled radioactive DNA, which has disadvantages known to anybody skilled in the art. Therefore, it was an object of the present invention to provide a faster method for determination of the base-sequence of nucleic acids.

This object has been achieved by copying the resolved product rows onto a stimulable phosphor sheet in the form of a radiation energy stored image and by scanning the phosphor sheet with an electromagnetic wave to leave at least a portion of radiation energy stored in said phosphor sheet as stimulated emission to detect locational information on the resolved radioactively labeled cleavage products.

Brief description of drawings

Figure 1 is a schematic view of an autoradiograph of resolved rows given by resolution of a set of radioactively labeled DNA base specific cleavage products and a mixture thereof on a support medium.

Figure 2 shows an example of the read-out system for reading out the locational information on radioactively labeled products copied from the sample and stored in a stimulable phosphor sheet employed in the embodiment of the present invention.

Detailed description of the invention

There is no limitation on DNA or DNA fragment to be sequenced according to the present invention, and examples of DNA and DNA fragment include a variety of DNA, fragments of DNA specifically cleaved at an optional position thereof, as well as their derivatives. The arts relating to the procedure for labeling DNA or DNA fragment or to the procedure for obtaining the radioactively labeled base specific cleavage products by applying a base specific cleaving reaction to the radioactively labeled DNA or DNA fragment is known. These arts are employable in the present invention. For instance, the representative arts on the above-mentioned procedures are briefly given in: "Reading the genetic information in the original language: A surprising method for sequencing the bases of DNA" written in Japanese by Kin-ichiro Miura, Modern Chemistry, September 1977, pp. 46—54 (Tokyo Kagaku Dozin Ltd., Japan).

More detailed description on the procedure is given in: METHODS IN ENZYMOLOGY, VOL. 65, PART I (ACADEMIC PRESS, NEW YORK, LONDON, TORONTO, SYDNEY, SAN FRANCISCO, 1980).

Representing examples of the method for resol-

ving (or developing) the above-mentioned radioactively labeled products on a support mediu include an electrophoresis using one of various resolving medium such as a gel in the form of layer, column or the like, a molded polymer film such as cellulose diacetate film, and a filter paper, and a thin layer chromatography using a support medium of such as silica gel. Among these, the electrophoresis using a gel support medium is preferably employed in the present invention.

The method for determination of base sequence of DNA or DNA fragment according to the present invention is described hereinafter, utilizing the above-mentioned Maxim-Gilbert method and referring to a typical example using a combination of five base specific cleavage products (or mixtures) given below:

1) guanine (G) specific cleavage products
+adenine (A) specific cleavage products
+thymine (T) specific cleavage products
+cytosine (C) specific cleavage products.
2) guanine (G) specific cleavage products,
3) guanine (G) specific cleavage products
+adenine (A) specific cleavage products,
4) thymine (T) specific cleavage products
+cytosine (C) specific cleavage products,
and
5) cytosine (C) specific cleavage products.

The mixture 1) contains four kinds of the base specific cleavage products and is employed for forming a resolved row for reference.

DNA labeled with a radioactive element ($^{32}$P) is specifically cleaved at the constitutional base units by chemical reactions according to the conventional manner, respectively to prepare a set of the base specific cleavage products 2) through 5), in which the cleavage products are radioactively labeled. Some of these cleavage products are appropriately mixed to prepare the radioactively labeled base specific cleavage product mixture 1).

The cleavage product mixture 1) and respectively cleavage products 2) through 5) are resolved in parallel by electrophoresis on one gel support medium in such manner that the mixture 1) is resolved in the center, to produce five rows of the resolved cleavage products (the resolved rows are not visible).

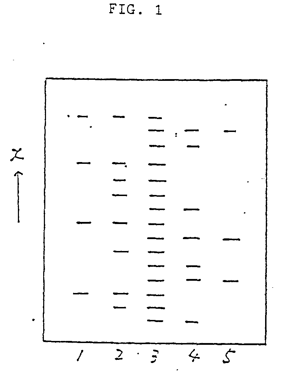

Figure 1 shows a drawing of an image obtained by autoradiography of five resolved rows of the radioactively labeled base specific cleavage products and their mixtures:

1) a resolved row indicating resolved positions of the guanine (G) specific cleavage products,

2) a resolved row indicating resolved positions of both the guanine (G) specific cleavage products and the adenine (A) specific cleavage products,

3) a resolved row indicating resolved positions of the thymine (T) specific cleavage products, the cytosine (C) specific cleavage products, the guanine (G) specific cleavage products, and the adenine (A) specific cleavage products,

4) a resolved row indicating resolved positions of both the thymine (T) specific cleavage products and the cytosine (C) specific cleavage products, and

5) a resolved row indicating resolved positions of the cytosine (C) specific cleavage products.

The third row, i.e., above-mentioned row 3) is a resolved row indicating resolved positions of all base specific cleavage products (G, A, T, C) and is employed as an internal reference row (reference row) for determination of the base sequence.

Identification of the resolved cleavage products distributed in each of the resolved rows in the form of belts utilizing the internal reference row can be done, for instance, as follows:

In the first step, the third row (internal reference row) and the adjacent second row are compared therebetween. Since the internal reference row indicates the resolved positions of all kinds of the base specific cleavage products, all of the resolved positions of the (G) specific cleavage products and (A) specific cleavage products shown in the second row necessarily correspond to a part of the resolved positions shown in the internal reference row. Accordingly, an observed dislocation between the internal reference row and the second row can be correctly detected for identification by the comparison between all of the resolved positions in the second row and the corresponding positions in the internal reference row.

In the second step, the third row (internal reference row) and the adjacent fourth row are compared therebetween. Since the internal reference row indicates the resolved positions of all kinds of the base specific cleavage products, all of the resolved positions of the (T) specific cleavage products and (C) specific cleavage products shown in the fourth row necessarily correspond to a part of the resolved positions shown in the internal reference row. Further, since the resolved positions in the second row and fourth row are exclusive of each other, the resolved positions in the fourth row are more accurately identificated (or assigned) utilizing said exclusive relation. Accordingly, an observed dislocation between the internal reference and the fourth row can be correctly detected for identification by the comparison between all of the resolved positions in the fourth row and the corresponding positions in the internal reference row.

In the final steps, the resolved positions of (G) specific cleavage products and (A) specific cleavage products are respectively identified by comparison between the second resolved row and the first resolved row. Likewise, the resolved positions of (T) specific cleavage products and (C) specific cleavage products are respectively identified by comparison between the fourth resolved row and the fifth resolved row. In these comparison procedures for identification of the resolved positions of the base specific cleavage products, the degree of dislocation of resolved positions detected therein can be taken into consideration for other comparison to make the comparison and identification easy and accurate.

Accordingly, the method of determination of base sequence of DNA or DNA fragment exemplified as above makes the identification of the base specific cleavage products more easy and accurate in comparison with the conventional method involving the resolution bf four base specific cleavage products groups (G, G+A, T+C, C) or the resolution of the simple set of four base specific cleavage products groups (G, A, T, C).

In the above description, the resolution is described in the example using the internal reference row resolved in the center among the other rows. However, the internal reference row can be arranged in an optional position.

Moreover, the internal reference row can be arranged plurally. For instance, if the internal reference row and the other row can be arranged alternately, the identification of the resolved positions of each base specific cleavage products is made more easy and accurate.

The above-described combination of G+A+T+C, G, A+G, T+C, and C is one example of the combinations of the base specific cleavage products employable in the method for determination of base sequence of DNA or DNA fragment of the present invention. Other various combinations can be also employable for performing the base sequencing in a substantially same manner as in the above.

Furthermore, although the above description is given in the arrangement of five cleavage products-resolved rows respectively distributed on a support medium in one dimensional directions, the number of the resolved rows are not restricted thereto. The rows can be in a number less than or more than five.

Accordingly, the determination of base sequence of DNA or DNA fragment includes the determination of all base sequence as well as the determination of sequence of a part of the constitutional bases as exemplified above.

In addition, although the description given hereinbefore is concerned with an embodiment in which the determination of base sequence is performed on one kind of DNA or DNA fragment on a support medium, a single support medium can be employed for simultaneous determinations of base sequences of a number of DNA or DNA fragments.

According to the present invention, the method for determination of base sequence of DNA or DNA fragment is performed utilizing a radiation image recording and reproducing method in which a stimulable phosphor sheet is employed.

The stimulable phosphor sheet employable in the present invention has been alternatively mentioned in the name of a radiation image storage panel, and described in, for instance, U.S. Patent No. 4,239,968. Accordingly, the general constitution of the stimulable phosphor sheet is already known. The stimulable phosphor sheet is used to record and reproduce the image produced by the radiation energy having passed through an object or having been radiated by an object.

The radiation image recording and reproducing process comprises steps of: causing the stimulable phosphor of the phosphor sheet to absorb a radiation energy having passed through an object or having been radiated by an object; exciting the stimulable phosphor with an electromagnetic wave such as visible light or infrared rays (hereinafter referred to as "stimulating rays") to sequentially release the radiation energy stored in the stimulable phosphor as light emission; photoelectrically detecting the emitted light to give an electric signal; and reproducing the electric signal in the form of a visible image on a recording material such as a photosensitive film or on a display device.

The stimulable phosphor sheet preferably employed in the autoradiography of the present invention is described briefly in the following.

The stimulable phosphor sheet has a basic structure comprising a substrate and a phosphor layer provided on one surface of the substrate. Further, a transparent film is generally provided on the free surface (surface not facing the substrate) of the phosphor layer to keep the phosphor layer from chemical deterioration or physical shock.

The phosphor layer comprises a binder and a simulable phosphor dispersed therein. When excited with an electromagnetic wave such as visible light or infrared rays after having been exposed to a radiation, the stimulable phosphor emits light (stimulated emission). Accordingly, a radiation having been radiated from a sample containing the radioactively labeled substances, for instance, is absorbed by the phosphor layer of the stimulable phosphor sheet in proportion to the radiation dose, and a radiation image of the sample is stored in the stimulable phosphor sheet in the form of a radiation energy stored image. The stored image can be released as stimulated emission (light emission) by applying an electromagnetic wave such as visible light or infrared rays (stimulating rays) onto the stimulable phosphor sheet. The stimulated emission is then photoelectrically detected to convert it to electric signals, and thus the radiation energy stored image can be converted to a visible image or numeral and/or symbol which represent the locational information on the radioactive substance, namely, the radioacty labeled substance.

A material of the substrate of the stimulable phosphor sheet employed in the present invention can be selected from those employed in the conventional radiographic intensifying screens. Examples of the substrate material include plastic films such as films of cellulose acetate, polyester, polyethylene terephthalate, polyamide, polyimide, triacetate and polycarbonate; metal sheets such as aluminum foil and aluminum alloy foil; ordinary papers; baryta paper; resin-coated papers; pigment papers containing titanium dioxide or the like; and papers sized with polyvinyl alcohol or the like. From a viewpoint of characteristics of a stimulable phosphor sheet as the information recording material, a plastic film is preferably employed as the substrate material of the invention. The plastic film may contain a light-absorbing material such as carbon black, or may contain a light-reflecting material such as titanium dioxide. The former is appropriate for a high sharpness type stimulable phosphor sheet, while the latter is appropriate for a high sensitivity type stimulable phosphor sheet.

In the preparation of a known stimulable phosphor sheet, one or more additional layers are occasionally provided between the substrate and the phosphor layer, so as to enhance the adhesion between the substrate and the phosphor layer, or to improve the sensitivity of the sheet or the quality of an image provided thereby. For instance, a subbing layer or an adhesive layer may be provided by coating a polymer material such as gelatin over the surface of the substrate on the phosphor layer side. Otherwise, a light-reflecting layer or a light-absorbing layer may be provided by forming a polymer material layer containing a light-reflecting material such as titanium dioxide or a light-absorbing material such as carbon black. One or more of these additional layers may be provided depending on the type of the stimulable phosphor sheet to be obtained.

As described in Japanese Patent Application No. 57(1982)—82431 (which corresponds to U.S. Patent Application No. 496,278 and the whole content of which is described in European Patent Publication No. 92241), the phosphor layer side surface of the substrate (or the surface of an adhesive layer, light-reflecting layer, or light-absorbing layer in the case where such layers provided on the phosphor layer) may be provided with protruded and depressed portions for enhancement of the sharpness of radiographic image, and the constitution of those protruded and depressed portions can be selected depending on the purpose of the stimulable phosphor sheet to be prepared.

Onto the above-mentioned substrate, a phosphor layer is provided. The phosphor layer comprises basically a binder and stimulable phosphor particles dispersed therein.

The stimulable phosphor, as described hereinbefore, gives stimulated emission when excited by stimulating rays after exposure to a radiation. From the viewpoint of practical use, the stimulable phosphor is desired to give stimulated emission in the wavelength region of 300—500 nm when excited by stimulating rays in the wavelength region of 400—850 nm.

Examples of the stimulable phosphor employable in the stimulable phosphor sheet utilized in the present invention include:

SrS:Ce, Sm, SrS:Eu, Sm, $ThO_2$:Er, and $La_2O_2S$:Eu, Sm, as describd in U. S. Patent No. 3,859,527; ZnS:Cu, Pb, $BaO \cdot xAl_2O_3$:Eu, in which $x$ is a number satisfying the condition of $0.8 \leqq x \leqq 10$, and $M^{2+}O \cdot xSiO_2$:A, in which $M^{2+}$ is at least one divalent metal selected from the group consisting of Mg, Ca, Sr, Zn, Cd and Ba, A is at least one element selected from the group consisting of Ce, Tb, Eu, Tm, Pb, Tl, Bi and Mn, and $x$ is a number satisfying the condition of $0.5 \leqq x \leqq 2.5$, as described in U.S. Patent No. 4,326,078;

$(Ba_{1-x-y}, Mg_x, Ca_y)FX$:a$Eu^{2+}$, in which X is at least one element selected from the group consisting of Cl and Br, $x$ and $y$ are numbers satisfying the conditions of $0<x+y\leqq0.6$, and $xy\neq0$, and $a$ is a number satisfying the condition of $10^{-6}\leqq a\leqq5\times10^{-2}$, as described in Japanese Patent Provisional Publication No. 55(1980)-12143;

LnOX:xA, in which Ln is at least one element selected from the group consisting of La, Y, Gd and Lu, X is at least one element selected from the group consisting of Cl and Br, A is at least one element selected from the group consisting of Ce and Tb, and $x$ is a number satisfying the condition of $0<x<0.1$, as described in the above-mentioned U.S. Patent No. 4,236,078;

$(Ba_{1-x},\ M^{II}{}_x)FX:yA$, in which $M^{II}$ is at least one divalent metal selected from the group consisting of Mg, Ca, Sr, Zn and Cd, X is at least one element selected from the group consisting of Cl, Br and I, A is at least one element selected from the group consisting of Eu, Tb, Ce, Tm, Dy, Pr, Ho, Nd, Yb and Er, and $x$ and $y$ are numbers satisfying the conditions of $0\leqq x\leqq0.6$ and $0\leqq y\leqq0.2$, respectively, as described in Japanese Patent Provisional Publication No. 55(1980)-12145;

$M^{II}FX\cdot xA:yLn$, in which $M^{II}$ is at least one element selected from the group consisting of Ba, Ca, Sr, Mg, Zn and Cd; A is at least one compound selected from the group consisting of BeO, MgO, CaO, SrO, BaO, ZnO, $Al_2O_3$, $Y_2O_3$, $La_2O_3$, $In_2O_3$, $SiO_2$, $TiO_2$, $ZrO_2$, $GeO_2$, $SnO_2$, $Nb_2O_5$, $Ta_2O_5$ and $ThO_2$; Ln is at least one element selected from the group consisting of Eu, Tb, Ce, Tm, Dy, Pr, Ho, Nd, Yb, Er, Sm and Gd; X is at least one element selected from the group consisting of Cl, Br and I; and $x$ and $y$ are numbers satisfying the conditions of $5\times10^{-5}\leqq x\leqq0.5$ and $0<y\leqq0.2$, respectively, as described in Japanese Patent Provisional Publication No. 55(1980)-160078;

$(Ba_{1-x},\ M^{II}{}_x)F_2\cdot aBaX_2:yEu,\ zA$, in which $M^{II}$ is at least one element selected from the group consisting of Be, Mg, Ca, Sr, Zn and Cd; X is at least one element selected from the group consisting of Cl, Br and I; A is at least one element selected from the group consisting of Zr and Sc; and $a$, $x$, $y$ and $z$ are numbers satisfying the conditions of $0.5\leqq a\leqq1.25$, $0\leqq x\leqq1$, $10^{-6}\leqq y\leqq2\times10^{-1}$, and $0<z\leqq10^{-2}$, respectively, as described in Japanese Patent Provisional Publication No. 56(1981)-116777;

$(Ba_{1-x},\ M^{II}{}_x)F_2\cdot aBaX_2:yEu,\ zB$, in which $M^{II}$ is at least one element selected from the group consisting of Be, Mg, Ca, Sr, Zn and Cd; X is at least one element selected from the group consisting of Cl, Br and I; and $a$, $x$, $y$ and $z$ are numbers satisfying the conditions of $0.5\leqq a\leqq1.25$, $0\leqq x\leqq1$, $10^{-6}\leqq y\leqq2\times10^{-1}$, and $0<z\leqq2\times10^{-1}$, respectively, as described in Japanese Patent Provisional Publication No. 57(1982)-23673;

$(Ba_{1-x},\ M^{II}{}_x)F_2\cdot aBaX_2:yEu,\ zA$, in which $M^{II}$ is at least one element selected from the group consisting of Be, Mg, Ca, Sr, Zn and Cd; X is at least one element selected from the group consisting of Cl, Br and I; A is at least one element selected from the group consisting of As and Si; and $a$, $x$, $y$ and $z$ are numbers satisfying the conditions of $0.5\leqq a\leqq1.25$, $0\leqq x\leqq1$, $10^{-6}\leqq y\leqq2\times10^{-1}$, and $0<z\leqq5\times10^{-1}$, respectively, as described in Japanese Patent Provisional Publication No. 57(1982)-23675;

$M^{III}OX:xCe$, in which $M^{III}$ is at least one trivalent metal selected from the group consisting of Pr, Nd, Pm, Sm, Eu, Tb, Dy, Ho, Er, Tm, Yb, and Bi; X is at least one element selected from the group consisting of Cl and Br; and $x$ is a number satisfying the condition of $0<x<0.1$, as described in Japanese Patent Application No. 56(1981)-167498;

$Ba_{1-x}M_{x/2}L_{x/2}FX:yEu^{2+}$, in which M is at least one alkali metal selected from the group consisting of Li, Na, K, Rb and Cs; L is at least one trivalent metal selected from the group consisting of Sc, Y, La, Ce, Pr, Nd, Pm, Sm, Gd, Tb, Dy, Ho, Er, Tm, Yb, Lu, Al, Ga, In and Tl; X is at least one halogen selected from the group consisting of Cl, Br and I; and $x$ and $y$ are numbers satisfying the conditions of $10^{-2}\leqq x\leqq0.5$ and $0<y\leqq0.1$, respectively, as described in Japanese Patent Application No. 57(1982)-89875;

$BaFX\cdot xA:yEu^{2+}$, in which X is at least one halogen selected from the group consisting of Cl, Br and I; A is at least one fired product of a tetrafluoro boric acid compound; and $x$ and $y$ are numbers satisfying the conditions of $10^{-6}\leqq x\leqq0.1$ and $0<y\leqq0.1$, respectively, as described in Japanese Patent Application No. 57(1982)-137374;

$BaFX\cdot xA:yEu^{2+}$, in which X is at least one halogen selected from the group consisting of Cl, Br and I; A is at least one fired product of a hexafluoro compound selected from the group consisting of monovalent and divalent metal salts of hexafluoro silicic acid, hexafluoro titanic acid and hexafluoro zirconic acid; and $x$ and $y$ are numbers satisfying the conditions of $10^{-6}\leqq x\leqq0.1$ and $0<y\leqq0.1$, respectively, as described in Japanese Patent Application No. 57(1982)-158048;

$BaFX\cdot nNaX':aEu^{2+}$, in which each of X and X' is at least one halogen selected from the group consisting of Cl, Br and I; and $x$ and $a$ are numbers satisfying the conditions of $0<x\leqq2$ and $0<a\leqq0.2$, respectively, as described in Japanese Patent Application No. 57(1982)-166320;

$M^{II}FX\cdot xNaX':yEu^{2+}:zA$, in which $M^{II}$ is at least one alkaline earth metal selected from the group consisting of Ba, Sr and Ca; each of X and X' is at least one halogen selected from the group consisting of Cl, Br and I; A is at least one transition metal selected from the group consisting of V, Cr, Mn, Fe, Co and Ni; and $x$, $y$ and $z$ are numbers satisfying the conditions of $0<x\leqq2$, $0<y\leqq0.2$ and $0<z\leqq10^{-2}$, respectively, as described in Japanese Patent Application No. 57(1982)-166696; and

$M^{II}FX\cdot aM^IX'\cdot bM^{II}X''_2\cdot cM^{III}X'''_3\cdot xA:yEu^{2+}$, in which $M^{II}$ is at least one alkaline earth metal selected from the group consisting of Ba, Sr and Ca; $M^I$ is at least one alkali metal selected from the group consisting of Li, Na, K, Rb and Cs; $M^{II}$ is at least one divalent metal selected from the group consisting of Be and Mg; $M^{III}$ is at least one trivalent metal selected from the group consisting of Al, Ga, In and Tl; A is at least one metal oxide;

X is at least one halogen selected from the group consisting of Cl, Br and I; each of X', X'' and X''' is at least one halogen selected from the group consisting of F, Cl, Br and I; $a$, $b$ and $c$ are numbers satisfying the conditions of $0 \leqq a \leqq 2$, $0 \leqq b \leqq 10^{-2}$, $0 \leqq c \leqq 10^{-2}$ and $a+b+c \geqq 10^{-6}$; and $x$ and $y$ are numbers satisfying the conditions of $0 < x \leqq 0.5$ and $0 < y \leqq 0.2$, respectively, as described in Japanese Patent Application No. 57(1982)-184455.

The above-described stimulable phosphor are given by no means to restrict the stimulable phosphor employable in the present invention. Any other phosphors can be also employed, provided that the phosphor gives stimulated emission when excited with stimulating rays after exposure to a radiation.

In carrying out the autoradiographic process of the present invention, a divalent europium-activated alkaline earth metal fluorohalide stimulable phosphor is preferably employed.

Examples of the binder to be contained in the phosphor layer include: natural polymers such as proteins (e.g., gelatin), polysaccharides (e.g., dextran) and gum arabic; and synthetic polymers such as polyvinyl butyral, polyvinyl acetate, nitrocellulose, ethylcellulose, vinylidene chloride-vinyl chloride copolymer, polymethyl methacrylate, vinyl chloride-vinyl acetate copolymer, polyurethane, cellulose acetate butyrate, polyvinyl alcohol, and linear polyester. Particularly preferred are nitrocellulose, linear polyester, and a mixture of nitrocellulose and linear polyester.

The phosphor layer can be formed on the substrate, for instance, by the following procedure.

In the first place, stimulable phosphor particles and a binder are added to an appropriate solvent (for example, lower alcohol, chlorinated hydrocarbon, ketone, ester, or ether), and they they are mixed to prepare a homogeneous coating dispersion of the phosphor particles in the binder solution.

The ratio between the binder and the stimulable phosphor in the coating dispersion may be determined according to the characteristics of the aimed stimulable phosphor sheet and the nature of the phosphor employed. Generally, the ratio therebetween is within the range of from 1:1 to 1:100 (binder:phosphor, by weight), preferably from 1:8 to 1:40.

The coating dispersion may contain a dispersing agent to increase the dispersibility of the phosphor particles therein, and also contain a variety of additives such as a plasticizer for increasing the bonding between the binder and the phosphor particles in the phosphor layer. Examples of the dispersing agent include phthalic acid, stearic acid, caproic acid and a hydrophobic surface active agent. Examples of the plasticizer include phosphates such as triphenyl phosphate, tricresyl phosphate and diphenyl phosphate; phthalates such as diethyl phthalate and dimethoxyethyl phthalate; glycolates such as ethylphthalyl ethyl glycolate and butylphthalyl butyl glycolate; and polyesters of polyethylene glycols with aliphatic dicarboxylic acids such as polyester of triethylene glycol with adipic acid and polyester of diethylene glycol with succinic acid.

The coating dispersion containing the phosphor particles and the binder prdpared as described above is applied evenly to the surface of the substrate to form a layer of the coating dispersion. The coating procedure can be carried out by a conventional method such as a method using a doctor blade, a roll coater or a knife coater.

After applying the coating dispersion to the substrate, the coating dispersion is then heated slowly to dryness so as to complete the formation of a phosphor layer. The thickness of the phosphor layer varies depending upon the characteristics of the aimed stimulable phosphor sheet, the nature of the phosphor, the ratio between the binder and the phosphor, etc. Generally, the thickness of the phosphor layer is within a range of from 20 μm to 1 mm, preferably from 50 to 500 μm.

The phosphor layer can be provided onto the substrate by the methods other than that given in the above. For instance, the phosphor layer is initially prepared on a sheet (false substrate) such as a glass plate, a metal plate or a plastic sheet using the aforementioned coating dispersion and then thus prepared phosphor layer is overlaid on the genuine substrate by pressing or using an adhesive agent.

As described above, a protective film is preferably provided on the phosphor layer. The protective film is generally prepared from a transparent cellulose derivative such as cellulose acetate or nitrocellulose; or a transpatent synthetic polymer such as polymethyl methacrylate, polyvinyl butyral, polyvinyl formal, polycarbonate, polyvinyl acetate, vinyl chloride-vinyl acetate copolymer, polyethylene terephthalate, polyethylene, polyvinylidene chloride or polyamide. The protective film preferably has a thickness within the range of 0.1—100 μm, and more preferably within the range of 0.3—50 μm.

One embodiment of the present invention employs the stimulable phosphor sheet having the above-described constitution as the radiosensitive material in the autoradiography in place of the conventional radiographic film. The exposing procedure of the invention can be done by placing the stimulable phosphor sheet and a sample containing a radioactively labeled substance together in layers for a certain period of time so as to have at least a portion of a radiation emitted by the radioactively labeled substance in the sample absorbed by the stimulable phosphor sheet.

In carrying out the above-mentioned exposing procedure, the temporary combination of the sample with the stimulable phosphor sheet is generally done by placing the sample in close contact with the phosphor sheet, but the contact is not required to be so close, and the exposure can be accomplished by keeping the phosphor sheet in a position adjacent to the sample.

The exposure time varies depending on the radioactivity of the radioactively labeled substance in the sample, the concentration of said substance, the sensitivity of the stimulable phosphor sheet, and the distance between the sample and the stimulable phosphor sheet, but in general the exposure is required to be done for a certain period of time, for instance, more than several seconds. In the case of employing the stimulable phosphor sheet as the radiosensitive material, however, the exposure time can be greatly reduced as compared with the exposure time required in the case employing the conventional radiographic film. Further, a precise control of the exposure time is not required in the case of employing the stimulable phosphor sheet, because the locational information on the radioactively labeled substances in the sample which has been copied from the sample through the exposing procedure can be electrically processed depending upon intensity of radiation energy emitted thereby, and distribution of the stored energy.

There is no specific limitation on the temperature for carrying out the exposing procedure, but it is advantageously characteristic aspect attached to the employment of the stimulable phosphor sheet in the autoradiography according to the present invention, that the exposure can be performed at an ambient temperature such as a temperature within 10—35°C. The exposure may be carried out, however, even at a low temperature, for instance, in the vicinity of 5°C or lower as adopted in the conventional autoradiography.

A method for reading out the locational information on the radioactively labeled substances in the sample copied and stored in the stimulable phosphor sheet according to the invention will be described briefly, referring to an embodiment of a read-out system shown in Figure 1 of the accompanying drawings.

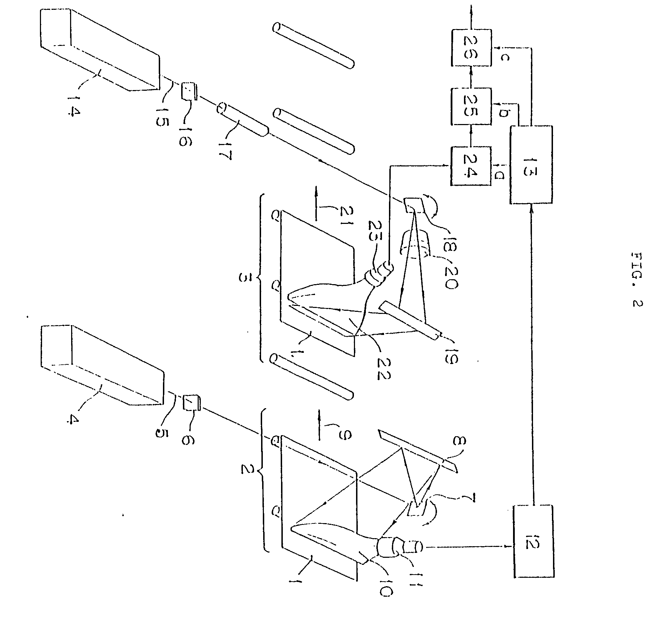

Figure 2 schematically illustrates an embodiment of the read-out system comprising a preliminary read-out section 2 for preliminarily reading out the one or two dimensional information on the location of the radioactively labeled substances stored (or recorded) in the stimulable phosphor sheet 1 (from which the sample generally has been removed, the stimulable phosphor sheet is hereinafter referred to as "phosphor sheet"), and a final read-out section 3 for finally reading out the desired locational information on the radioactively labeled substance stored in the phosphor sheet 1.

In the preliminary read-out section 2, the preliminary read-out operation is carried out in the following manner.

Laser beam 5 generated by a laser source 4 first passes through a filter 6 to cut off a light beam in the wavelength region corresponding to the wavelength region of stimulated emission to be emitted from the phosphor sheet 1 in response to stimulation with the laser beam 5. The laser beam 5 is subsequently deflected by a beam deflector 7 such as a galvanometer mirror, and reflected by a plane reflecting mirror 8. The deflected beam then impinges upon the phosphor sheet 1. The laser source 4 used herein is so selected as to avoid overlapping of the wavelength region of the laser beam 5 with the main wavelength region of the stimulated emission to be emitted from the phosphor sheet 1.

The phosphor sheet 1 is transferred to the direction along the arrow 9 under the irradiation of the above-mentioned deflected laser beam. Therefore, the whole surface of the phosphor sheet 1 is subjected to the irradiation of the deflected laser beam. The power of the laser beam 5 employed in the preliminary read-out section is adjusted to be lower than the power of the laser beam to be employed in the final read-out section by controlling the output of the laser source 4, the beam diameter of the laser beam 5, the scanning speed of the laser beam 5, and the transferring speed of the phosphor sheet 1.

When irradiated with the above-mentioned laser beam, the phosphor sheet 1 gives the stimulated emission having the emission intensity proportional to the radiation energy stored (or recorded) therein. The emission then enters into a light guiding sheet 10 for the preliminary readout. The light guiding sheet 10 has a linear edge face for receiving the emission, and the edge face is so positioned in the vicinity of the phosphor sheet as to correspond to the scanning line on the phosphor sheet 1. The exit of the light guiding sheet 10 is in the form of a ring and is connected to an light-receiving face of a light detector 11 such as a photomultiplier. The light guiding sheet 10 is made, for instance, by processing a sheet of a transparent thermoplastic resin such as an acrylic synthetic resin, and so constituted that the emission introduced from the linear edge face is transmitted to the exit under repeated total reflection within the sheet 10. The stimulated emission from the phosphor sheet 1 is guided in the interior of the light guiding sheet 10 to the exit, and received by the light detector 11.

The preferable shape and material of the light guiding sheet is disclosed in Japanese Patent Provisional Publications No. 55(1980)-87970 and No. 56(1981)-11397, etc.

On the light-receiving face of the light detector 11 is provided a filter which allows only the light of wavelength region of the stimulated emission to pass through and cuts off the light of the wavelength region of the stimulating rays (laser beam) so as to detect only the stimulated emission. The stimulated emission detected by the light detector 11 is converted to an electric signal, amplified in an amplifier 12 and transmitted to the output. The stored information output from the amplifier 12 is supplied to a control circuit 13 of the final read-out section 3. The control circuit 13 provides an amlification degree setting value $a$, a scale factor setting value $b$, and an image processing condition setting value $c$ so that a well readable image having even concentration and contrast can be obtained regardless of variation of the detected information.

The phosphor sheet 1 having been subjected to the preliminary read-out in the above-described manner is then transferred to the final read-out section 3.

In the final read-out section 3, the following read-out operation is performed.

The laser beam 15 generated by a laser source 14 for the final read-out passes through a filter 16 having the same function as that of the above-mentioned filter 6, and then the beam diameter is precisely adjusted in a beam expander 17. Subsequently, the laser beam is deflected by a beam deflector 18 such as a galvanometer mirror, and reflected by a plane reflection mirror 19. The deflected beam then impinges one-dimensionally upon the phosphor sheet 1. Between the beam deflector 18 and the plane reflection mirror 19 a f$\theta$ lens 20 is provided so that the beam speed is continuously kept constant when the deflected laser beam is scanned on the phosphor sheet 1.

The phosphor sheet 1 is transferred in the direction along the arrow 21 under the irradiation with the above-mentioned deflected laser beam. Accordingly, the whole surface of the phosphor sheet is subjected to the irradiation in the same manner as in the preliminary read-out operation.

When irradiated with the above-mentioned laser beam, the phosphor sheet 1 gives the stimulated emission in proportion to the radiation energy stored therein in the same manner as in the preliminary read-out operation. The emission then enters into a light guiding sheet 22 for the final read-out. The light guiding sheet 22 for the final read-out is of the same material and has the same constitution as the light guiding sheet 10 employed for the preliminary read-out. The stimulated emission received is guided in the interior of the light guiding sheet 22 up to the exit under repeated total reflection, and then received by a light detector 23. On the light-receiving face of the light detector 23 is provided a filter which allows only the light of wavelength region of the stimulated emission to pass through and cuts off the light of the wavelength region of the stimulating rays (laser beam) so as to detect only the stimulated emission. The stimulated emission detected by the light detector 23 is converted to an electric signal, amplified to an electric signal adjusted to an appropriate level in an amplifier 24 according to the aforementioned amplification degree setting value $a$ and transmitted to an A/D converter 25. The adjusted electric signal is then converted to a digital signal in the A/D converter 25 according to an appropriate scale factor defined by the scale factor setting value $b$, and supplied to a signal processing circuit 26. In the circuit 26, the digital signal is so processed according to the image processing condition setting value $c$ as to give a well readable visible image having well adjusted concentration and contrast, and then transmitted to a recording device (not shown), optionally upon storage in a storing means such as a magnetic tape.

Various recording devices based on various systems can be employed for the above-described purpose, for instance, a device for visualizing optically by scanning on a lightsensitive material with laser beam, etc., a display means for visualizing electrically on CRT, etc., a means for printing a radiation image displayed on CRT by means of a video printer, and a means for visualizing on heat-sensitive recording material using thermic rays.

The recording device employable in the invention is not restricted to the visualizing devices such as above, and the one or two dimensional information on the location of the radioactively labeled substances in a sample may be recorded or produced, for instance, in the form of numeral and/or symbol.

In the above description on the method for reading out the locational information on the radioactively labeled substance copied and stored in the stimulable phosphor sheet, a read-out operation involving both the preliminary read-out operation and the final read-out operation has been given. However, the read-out operation employable in the present invention is not limited to the above-described embodiment. For instance, the preliminary read-out operation may be omitted if the content of the radioactive substance in the sample and an adequate exposure time for the sample is previously known.

Further, it is natural that other suitable methods than the above-mentioned embodiments may be used for reading out the locational information on the radioactively labeled substances copied from the sample and stored in the stimulable phosphor sheet.

In the present invention, the term "locational information" of the radioactively labeled substances means to include a variety of information relating to the location of the radioactively labeled substances, or the aggregation thereof, being present in the sample, such as the location, the shape, the concentration, the distribution combination of thereof.

The determination of base sequence of DNA or DNA fragment can be performed in the same manner as described in the base sequencing method utilizing the conventional autoradiography in which the radiographic film is employed, except that the rows of the resolved base specific cleavage products are copied onto the stimulable phosphor sheet in the form of a radiation energy image and subsequently said phosphor sheet is scanned with an electromagnetic wave to release at least a portion of radiation energy stored in said phosphor sheet as stimulated emission to detect locational information on the resolved radioactively labeled cleavage products.

The determination of base sequence of the DNA or DNA fragment can be made based on said locational information detected in the above.

The method for determination of base sequence of DNA or DNA fragment utilizing the above-described radiation image recording and reproducing method employing a stimulable phosphor sheet is more advantageous than the method utilizing the conventional autoradiography employing a radiographic film, because the procedure for copying the resolved rows onto the stimulable phosphor sheet can be done at an ambient temperature within a prominently shorter period of time in the former method. Moreover, in the former method employing a stimulable phosphor sheet, the locational information on the resolved rows is obtained as digital signal. Accordingly, the digital signal can be processed in a computer to give the base sequence of DNA or DNA fragment under analysis directly with no visualization of the autoradiograph of the resolved rows. Morever, the noises introduced into the phosphor sheet in the course of the copying procedure can be removed through the processing to make it possible more accurate base sequencing.

## Claims

1. A method for determination of the base sequence of DNA or DNA fragments comprising the following steps:

(a) resolving radioactively labeled base specific cleavage products obtained by base specific cleavage of a radioactively labeled DNA or DNA fragment on a support medium to produce at least one resolved cleavage product row;

said step comprises simultaneously resolving a reference mixture of cleavage products of the DNA or DNA fragment comprising four kinds of radioactively labeled cleavage products obtained respectively by base-specifically cleaving radioactively labeled DNA or DNA fragments at different constitutional base positions on the same support medium;

(b) making the resolved product row visible;

(c) determining base sequence of the DNA or DNA fragment;

said step comprises comparing and identifying, by referring to the resolved positions of the reference mixture of cleavage products detected in a resolved row thereof, the resolved positions of the base specific cleavage products detected in the other resolved row thereof;

characterized in that the resolved product row is copied onto a stimulable phosphor sheet in the form of a radiation energy stored image and that said phosphor sheet is scanned with an electromagnetic wave to release at least a portion of radiation energy stored in said phosphor sheet as stimulated emission to detect locational information on the resolved radioactively labeled cleavage products.

2. The method as claimed in claim 1, in which the step of resolving both the radioactively labeled base specific cleavage products and the reference mixture of four kinds of the radioactively labeled base specific cleavage products is performed through electrophoresis on a gel support medium of polymer substance.

3. The method as claimed in claim 1 or 2, in which said reference mixture of four kinds of radioactively labeled base specific cleavage products comprises:

α) guanine specific cleavage products, adenine and guanine specific cleavage products, thymine specific cleavage products, and cytosine specific cleavage products, and

the radioactively labeled base specific cleavage products for formation of the other rows essentially consist of four product groups comprising independently the base specific cleavage products of:

β) guanine specific cleavage products,
γ) guanine specific cleavage products +adenine specific cleavage products
δ) thymine specific cleavage products +cytosine specific cleavage products, and
ε) cytosine specific cleavage products.

## Patentansprüche

1. Verfahren zur Ermittlung der Basensequenz von DNA oder DNA-Fragmenten, bei dem

a) radioaktiv markierte basenspezifische Spaltprodukte, die durch basenspezifische Spaltung von radioaktiv markierter DNA oder DNA-Fragmenten erhalten wurden, auf einem Trägermedium aufgelöst werden, um mindestens eine aufgelöste Spaltprodukt-Bande zu erhalten; wobei bei diesem Schritt gleichzeitig eine Referenzmischung aus Spaltprodukten von DNA oder DNA-Fragmenten, welche vier Arten von radioaktiv markierten Spaltprodukten umfaßt, die jeweils durch basenspezifische Spaltung an verschiedenen konstitutionellen Basenpositionen der radioaktiv marketern DNA oder DNA-Fragmenten erhalten wurden, auf dem gleichen Trägermedium aufgelöst werden,

b) die aufgelöste Produktbande sichtbar gemacht wird,

c) die Basensequenz der DNA oder des DNA-Fragments ermittelt wird, wobei bei diesem Schritt durch Zuordnen zu den aufgelösten Positionen der in einer aufgelösten Bande ermittelten Spaltprodukte der Referenzmischung, die aufgelösten Positionen der in der anderen aufgelösten Bande ermittelten basenspezifischen Spaltprodukte verglichen und identifiziert werden,

dadurch gekennzeichnet, daß die aufgelöste Produktbande auf eine stimulierbare Leuchtstoffschicht in Form eines Strahlungsenergie-Speicherbildes kopiert wird und daß diese Leuchtstoffschicht mit einer elektromagnetischen Welle abgetastet wird, um wenigstens einen Teil der in der Leuchtstoffschicht gespeicherten Strahlungsenergie als stimulierte Emission freizusetzen, um die Information der Lokalisation auf den aufgelösten radioaktiv markierten Spaltprodukte zu ermitteln.

2. Verfahren nach Anspruch 1, dadurch gekennzeichnet, daß die Auflösung der radioaktiv markierten basenspezifischen Spaltprodukte und der Referenzmischung mit vier Arten von radioaktiv markierten basenspezifischen Spaltprodukten durch Elektrophorese auf einem Gelträgermedium aus einer polymeren Substanz durchgeführt wird.

3. Verfahren nach Anspruch 1 oder 2, dadurch gekennzeichnet, daß die Referenzmischung aus vier Arten von radioaktiv markierten basenspezifischen Spaltprodukten

a) Guanin spezifische Spaltprodukte, Adenin und Guanin spezifische Spaltprodukte, Thymidin spezifische Spaltprodukte und Cytosin spezifische Spaltprodukte umfaßt,

und die radioaktiv markierten basenspezifischen Spaltprodukte zur Bildung der anderen Banden im wesentlichen aus vier Produktgruppen, die unabhängig die basenspezifischen Spaltprodukte

b) Guanin spezifische Spaltprodukte,

c) Guanin spezifische Spaltprodukte und Adenin spezifische Spaltprodukte,

d) Thymidin spezifische Spaltprodukte und Cytosin spezifische Spaltprodukte, und

e) Cytosin spezifische Spaltprodukte umfassen, bestehen.

## Revendications

1. Procédé de détermination de la séquence de bases du DNA ou de fragments de DNA comprenant les stades suivants:

a) résolution des produits de coupure spécifique des bases radioactivement marqués obtenus par coupure spécifique des bases d'un DNA ou d'un fragment de DNA radioactivement marqué sur un milieu de support pour produire au moins une rangée de produits de coupure résolus;

ce stade comprend la résolution simultanée d'un mélange de référence de produits de coupure du DNA ou de fragments de DNA comprenant quatre espèces de produits de coupure radioactivement marqués obtenus respectivement en coupant d'une manière spécifique aux bases un DNA ou des fragments de DNA radioactivement marqués à diverses positions des bases constitutionnelles sur le même milieu de support;

b) rendre visible la rangée de produits résolus,

c) détermination de la séquence de bases du DNA ou du fragment de DNA;

ce stade comprend la comparaison et l'identification, en se référant aux positions résolues du mélange de référence de produits de coupure détectés dans une rangée résolue de celui-ci, des positions résolues des produits de coupure spécifique des bases détectés dans l'autre rangée résolue de ceux-ci,

caractérisé en ce que la rangée de produits résolus est copiée sur une feuille de substance luminescente stimulable sous la forme d'une image stockée d'énergie de rayonnement et en ce que cette feuille de substance luminescente est balayé avec une onde électromagnétique pour libérer au moins une partie de l'énergie de rayonnement stockée dans cette feuille de substance luminescente sous la forme d'un émission stimulée pour détecter l'information concernant la localisation des produits de coupure radioactivement marqués résolus.

2. Procédé suivant la revendication 1, dans lequel le stade de résxguyion à la fois des produits de coupure spécifique des bases radioactivement marqués et du mélange de référence des quatre espèces de produits de coupure spécifique des bases radioactivement marqués est effectué par électrophorèse sur un milieu de support de gel de substance polymère.

3. Procédé suivant la revendication 1 ou 2, dans lequel ce mélange de référence de quatre espèces de produits de coupure spécifique des bases radioactivement marqués comprend:

α) des produits de coupure spécifique de la guanine, des produits de coupure spécifique de l'adénine et de la guanine, des produits de coupure spécifique de la thymine et des produits de coupure spécifique de la cytosine,

et les produits de coupure spécifique des bases radioactivement marqués pour la formation des autres rangées se composent essentiellement de quatre groupes de produits comprenant indépendamment les produits de coupure spécifique des bases suivants:

β) produits de coupure spécifique de la guanine,

γ) produits de coupure spécifique de la guanine plus produits de coupure spécifique de l'adénine,

δ) produits de coupure spécifique de la thymine plus produits de coupure spécifique de la cytosine, et

ε) produits de coupure spécifique de la cytosine.

FIG. 1

FIG. 2